(19) Europäisches Patentamt
European Patent Office
Office européen des brevets

(11) Publication number: 0 035 831
B1

(12) # EUROPEAN PATENT SPECIFICATION

(45) Date of publication of patent specification: 08.01.86

(21) Application number: 81300538.6

(22) Date of filing: 10.02.81

(51) Int. Cl.$^4$: C 12 N 15/00, C 12 N 1/32, C 12 N 1/20, A 23 K 1/00 // C12R1/01

(54) **Method for making genetically modified microorganisms.**

(30) Priority: 07.03.80 GB 8007849

(43) Date of publication of application:
16.09.81 Bulletin 81/37

(45) Publication of the grant of the patent:
08.01.86 Bulletin 86/02

(84) Designated Contracting States:
AT BE CH DE FR GB IT LI LU NL SE

(56) References cited:
EP-A-0 037 273
GB-A-2 003 926

BIOLOGICAL ABSTRACTS, vol. 66, no. 7, April 1, 1978, Philadelphia, US Abstract 39386 A.C.Y. CHANG et al. "Construction and characterization of amplifiable multicopy DNA cloning vehicles derived from the P15A cryptic miniplasmid"

(73) Proprietor: IMPERIAL CHEMICAL INDUSTRIES PLC
Imperial Chemical House Millbank
London SW1P 3JF (GB)

(72) Inventor: Worsey, Michael John
156 Chester Road Helsby
Via Warrington Cheshire (GB)
Inventor: Windass, John David
6 Nursery Hollow
Glen Parva Leicester (GB)

(74) Representative: Aufflick, James Neil et al
Imperial Chemical Industries PLC Legal
Department: Patents PO Box 6 Bessemer Road
Welwyn Garden City Herts, AL7 1HD (GB)

(56) References cited:
13th INTERNATIONAL TNO CONFERENCE BIOTECHNOLOGY A HIDDEN PAST, A SHINING FUTURE, Rotterdam, 27, 28 March 1980 by Netherlands Central Organisation for Applied Scientific Research TNO, The Hague P.H. SENIOR et al. "The ICI single cell protein process", pages 97-101

EP 0 035 831 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

NATURE, vol. 287, October 2, 1980 J.D. WINDASS et al. "Improved conversion of methanol to single cell protein by methylophilus methylotrophus", pages 396-401

SCIENTIFIC AMERICAN, vol. 242, February 1980 S.N. COHEN et al. "Transposable genetic elements", pages 36-45

**Description**

This invention relates to a method for making genetically modified microorganisms, to genetically modified microorganisms and to a process for the production of single cell protein (SCP) using such genetically modified microorganisms.

In our UK Patent No. 1370892 we describe and claim a process for the production of SCP in which bacteria belonging inter alia to the species *Methylophilus methylotrophus* (formerly named *Pseudomonas methylotropha*) are cultivated in a medium comprising methanol as a carbon source and appropriate inorganic nutrients. In such a process it is desirable that a high carbon conversion (i.e. conversion of substrate (methanol) carbon to cellular carbon) is achieved. Ammonia is a convenient nitrogen source for such a process.

In the production of SCP by microbiological assimilation of methanol and ammonia, two pathways for ammonia assimilation by microorganisms have been identified, namely

(a) direct (GDH) pathway—

$$\underset{\text{alpha keto glutarate}}{HO_2C(CH_2)_2CO\ CO_2H}+NH_3+NAD(P)H$$

$$\rightleftarrows$$

$$\underset{\text{glutamate}}{HO_2C(CH_2)_2\underset{\substack{|\\NH_2}}{CH}\ CO_2H}+NAD(P)$$

This reaction is catalysed by the enzyme glutamate dehydrogenase (GDH) and requires one mole of NAD(P)H, equivalent to 3 moles of ATP per mole of ammonia assimilated.

(b) 2-stage (GS-GUS) pathway—

$$\underset{\text{glutamate}}{HO_2C(CH_2)_2\underset{\substack{|\\NH_2}}{CH}\ CO_2H}+NH_3+ATP \rightleftarrows \underset{\text{glutamine}}{H_2NCO(CH_2)_2\underset{\substack{|\\NH_2}}{CH}\ CO_2H}+ADP+Pi$$

This reaction is catalysed by the enzyme glutamine synthetase (GS).

$$HO_2C(CH_2)_2\ CO\ CO_2H+H_2NCO(CH_2)_2\underset{\substack{|\\NH_2}}{CH}\ CO_2H+NAD(P)H \rightleftarrows 2HO_2C(CH_2)_2\underset{\substack{|\\NH_2}}{CH}\ CO_2H+NAD(P)$$

This reaction is catalysed by the enzyme glutamine glutamate aminotransferase (GUS) (also known as glutamate synthase and as GOGAT). This combination of reactions forming the two-stage pathway requires one mole of ATP for the first stage and the equivalent of 3 moles for the second stage, i.e. a total of 4 moles of ATP, per mole of ammonia assimilated.

The exact mechanism by which ATP is consumed is immaterial. However overall the greater consumption of ATP in the two-stage mechanism results in conversion of more of the methanol to carbon dioxide than in the direct mechanism.

We have found that *Methylophilus methylotrophus* lacks the GDH enzyme and assimilates ammonia by the two-stage pathway and that this pathway predominates amongst methanol-utilizing bacteria proposed to date for SCP production. In our published UK Patent Specification No. 2003926A we describe an improved process for the production of single cell protein using microorganisms such as *Methylophilus methylotrophus* which have been genetically modified by incorporation of the gene specifying ammonia assimilation by the GDH enzyme route. This process gives improved conversion of substrate carbon to cellular carbon.

We have now developed an improved method for introducing genetic material, such as the gene specifying ammonia assimilation by the GDH enzyme route, into a microorganism, thereby producing improved genetically modified strains of e.g. *Methylophilus methylotrophus* and an improved process for the production of SCP. In particular our improved genetic modification method provides a means whereby an increased amount of the gene specifying ammonia assimilation by the GDH route can be introduced into e.g. a strain of *Methylophilus methylotrophus* thereby increasing the amount of the GDH enzyme present in the microorganism and improving the efficiency of the ammonia assimilating pathway established in the microorganism.

Explanation of terms and symbols

In this specification the terms and symbols listed below have the meanings given:—

NAD(P)—nicotinamide adenine dinucleotide (phosphate), the co-factor of the GDH or GUS enzyme (and many other enzymes) in its oxidised form;

NAD(P)H—nicotinamide adenine dinucleotide (phosphate), the co-factor of the GDH or GUS enzyme (and many other enzymes) in its reduced form;

ATP—adenosine triphosphate;

ADP—adenosine diphosphate;

DNA—deoxyribonucleic acid—genetic material of most organisms;

Pi—phosphate ion;

*gdh* gene—the gene specifying glutamate dehydrogenase, the key enzyme for ammonia assimilation by the GDH enzyme route;

*glt* gene—the gene specifying glutamate synthase, the key enzyme for ammonia assimilation by the GUS enzyme route;

*glt* B—the gene specifying glutamate synthase, the key enzyme for ammonia assimilation by the GUS enzyme route, in *E. coli*;

*rif*$^r$—rifampicin resistance—other antibiotic resistances are expressed in similar manner;

*Tc*$^s$—tetracycline sensitivity—other antibiotic sensitivities are expressed in similar manner;

Mu *cts*—temperature sensitive Mu phage—Mu is a temperate phage;

*transposon*—a segment of DNA which is transportable from one replicon to another and which can attach itself into a plasmid and frequently confer properties such as antibiotic resistances thereon, example transposon Tn5.

According to the present invention we provide a method for making a genetically modified microorganism by incorporating DNA material comprising a *gdh* gene into a microorganism deficient in this gene, so that expression of the *gdh* gene product, i.e. the active GDH enzyme, occurs in the previously *gdh* gene-deficient microorganism which method comprises the steps of (a) introducing into a first microorganism having the *gdh* gene a plasmid vector for said gene, and (b) transferring said gene on said vector into a second microorganism. The second microorganism may be the *gdh* gene-deficient microorganism intended as the ultimate recipient of the DNA material or it may be an intermediate, the DNA material comprising the *gdh* gene being transferred via one or more intermediate microorganism(s) from the first microorganism to the *gdh* gene-deficient microorganism.

Further according to the invention we provide a method for making a genetically modified microorganism by transferring DNA material comprising the *gdh* gene from a donor microorganism having said gene to a recipient microorganism deficient therein wherein the DNA material is transferred into the recipient microorganism on a plasmid vector formed from a multicopy plasmid which plasmid, when introduced into the recipient microorganism, is capable of replicating itself to produce more than 3 copies of itself per cell. The method may comprise a plurality of steps involving intermediary microorganisms during transfer of the DNA material from the donor to the recipient microorganism.

Further according to the invention we provide a method for making a genetically modified microorganism by transferring DNA material comprising the *gdh* gene from a donor microorganism having said gene to a recipient microorganism deficient therein which comprises a step wherein the *gdh* gene containing DNA material is transferred on a DNA fragment having at its ends target sites for the *SalI* restriction enzyme.

The invention further provides new microorganisms produced by the method thereof and derivatives therefrom, processes for producing single cell protein and/or amino acids using such microorganisms, dead cells of such microorganisms and feed—or foodstuffs for animal or human consumption containing such dead cells.

The invention further and particularly provides the novel microorganisms *Methylophilus methylotrophus* strain NCIB No. 11585 and variants and mutants derived therefrom, processes for the production of single cell protein and/or amino acids using said strain, variants or mutants, dead cells and amino acids produced by such processes and feed—or foodstuffs for animal or human consumption containing said dead cells.

Preferably the method of the invention is used to introduce the *gdh* gene into gram-negative bacteria lacking this gene.

The microorganism *Methylophilus methylotrophus* strain NCIB No. 11585 was produced by the genetic modification of strain NCIB No. 10515 as described in Example 1 (strain 11585 is Modification 3). A culture of strain NCIB No. 11585 is deposited at the National Collection of Industrial Bacteria, Torrey Research Station, Aberdeen, Scotland, UK. The microbiological characteristics of NCIB No. 10515 and of the species *Methylophilus methylotrophus* generally are set out in our UK Patent Specification No. 1370892. The microbiological characteristics of strain NCIB No. 11585 are the same as those of strain NCIB No. 10515 except that strain NCIB No. 11585 is resistant to sulphonamide, streptomycin and kanamycin.

Microorganisms which can usefully be modified by the method of the invention to incorporate the *gdh* gene include the following bacteria:—

*Methylophilus methylotrophus*—cultures of which are deposited as follows:—

NCIB 10508—10515 and 10592—10596, all inclusive;

NRRL B 5352—B 5364 inclusive;

FRI 1215—1227 inclusive;

*Pseudomonas methylonica*, the characteristics of which are described in UK Specification No. 1451020 and a culture of which is deposited as follows:—

FRI 2247 and 2248

*Pseudomonas methanolica*—UK Specification No. 1352728—ATCC 21704;

*Pseudomonas sp.*—UK Specification No. 1326582—ATCC 21438 and 21439;

*Methylomonas methanolica*—UK Specification No. 1420264—NRRL B 5458;

*Pseudomonas utilis*—UK Specification No. 1444072—FRI 1690 and 1691;

*Pseudomonas inaudita*—UK Specification No. 1444072—FRI 1693 and 1694;

*Methylomonas clara*—USP 4166004—ATCC 31226.

*NB*:

NCIB is the National Collection of Industrial Bacteria, Aberdeen;

NRRL is the Collection maintained by the US Department of Agriculture, Peoria, Illinois;

ATCC is the American Type Culture Collection;

FRI is the Fermentation Research Institute, Japan.

Such microorganisms may be used in processes for the production of SCP wherein, after modification by the method of the invention, they are aerobically cultured in an aqueous medium containing methanol as a source of assimilable carbon, ammonia and/or ammonium ion at a concentration sufficient to maintain the GDH reaction mechanism, and other inorganic nutrients, followed by recovery of SCP from the culture medium.

The conditions of the process for producing SCP are conveniently as described in UK Specification Nos. 1370892 and 1451020, except that the total concentration of ammonia and ammonium ion is preferably maintained at above 0.5 mM in zones of maximum methanol concentration. As a result of the decreased consumption of ATP there is less overall conversion of methanol to carbon dioxide and consequently the process is less exothermic and requires less cooling to maintain the temperature at the preferred level in the range 34—45°C. For optimum economy the active microorganism predominantly and preferably exclusively metabolises ammonia by the GDH route.

In the method of the invention for introducing a *gdh* gene into a microorganism deficient in this gene any suitable microorganism may form a source for the *gdh* gene, gram negative bacteria being preferred. Suitable sources include strains of *E. coli, Pseudomonas* and *Klebsiella*, although in general terms there is no a priori reason for selecting any particular gram negative bacterium as the source of the *gdh* gene. However *E. coli* is a well-known species whose properties have been widely researched. Hence although a wide variety of microorganisms could serve a sources of the *gdh* gene it is generally convenient to use *E. coli* for this purpose. In the particular instance where the *gdh* gene is to be transferred into a strain of *Methylophilus methylotrophus, E. coli* is further preferred as a source since we have found that the *E. coli gdh* gene can provide a functional GDH pathway in *Methylophilus methylotrophus* without any further manipulation of the gene.

In the method of the invention for the transfer of the *gdh* gene a fragment of DNA comprising the *gdh* gene is transferred using one or a succession of plasmid vectors from a microorganism having the *gdh* gene into a microorganism lacking said gene. In its simplest form the method can consist in introducing into the source microorganism for the *gdh* gene a plasmid which can act as a vector for the gene, mobilising the gene-containing DNA fragment onto the vector and transferring the gene-carrying vector into the microorganism lacking the *gdh* gene. However many variations and elaborations of this simple sequence are possible, one being described in detail in Example 1. For instance the plasmid used to take the gene out of the source microorganism may not be readily transferable into the recipient microorganism and it may therefore be convenient to introduce an extra step wherein the gene is transferred from the original plasmid vector onto another plasmid more readily transferable into the recipient microorganism. Such transfers may be effected by any suitable means, conveniently by use of appropriate restriction enzymes and ligases. In another instance the original plasmid vector may be large and may carry a considerable quantity of extraneous DNA making it difficult to manipulate. In this case it is convenient to transfer the gene-carrying DNA fragment onto a smaller plasmid. A suitable sequence of steps for transferring *gdh* gene from an *E. coli* strain into a strain of *Methylophilus methylotrophus* is described in Example 1. The number and exact nature of the steps required will vary in transfers involving different microorganisms depending upon a number of factors including the source and recipient microorganisms concerned and the availability of suitable plasmids and upon general convenience.

The plasmids used can be derived from any source. Suitable sources include *E. coli, Pseudomonas* and *Klebsiella*. Useful plasmids include those of the "P' incompatibility group, for example RP4, which are freely transmissible amongst a variety of gram negative bacteria. Other suitable plasmids for use during the transfer sequence include R300B, pRP301 and pACYC184. Preferably the plasmids are ones which are capable of transfer into the recipient microorganism. To facilitate transfer of the gene, e.g. to improve the

utility of a plasmid as a vector or to render it capable of transfer into a recipient microorganism, the properties of a plasmid may be modified by associating with it another DNA fragment or fragments. Examples of such other fragments include temperate phages such as Mu and λ phages and transposons such as Tn 5. Such phages and transposons can play a useful part in the transfer of the *gdh* gene. Temperature sensitive Mu phage (Mu *cts*) is useful in the preparation of the plasmid vector used to take the *gdh* gene from the source microorganism. When a microorganism containing (Mu *cts*) is grown at a partially inducing temperature transposition of Mu and adjacent genome DNA occurs without further maturation of phage particles. This transposition then facilitates transfer of fragments of the microorganism's DNA onto a plasmid such as RP4 present in the microorganism leading to a recombinant RP4 containing segments of genome DNA. Temperate phages and transposons can also be used to provide marker antibiotic resistances and target sites for restriction enzymes on otherwise deficient plasmids. For example transposon Tn 5 is very useful as a supplier of target sites for the *SalI* restriction enzyme to plasmids such as R300B, one such variant of R300B:Tn 5 is pTB70.

Groups of plasmids which are readily transferable into strain NCIB No. 1015 and are thus very useful in the invention are the Inc Q, Inc P and Inc W groups of plasmids. Included in the Inc Q group are plasmids R300B and other plasmids described by P. T. Barth and N. J. Grinter (J. Bacteriol., (1974), *120*, 618—30) and plasmid R1162. Included in the Inc P group are plasmids RP4 (described by N. Datta et al, J. Bacteriol., (1971), *108*, 1244—49), pRP 301 (described by P. T. Barth at pages 399—410 of "Plasmids of Medical, Environmental and Commercial Importance", Editors K. N. Timmis and A. Pühler, Published Elsevier, North Holland, 1979) and R 68.45. Included in the Inc W group are plasmids R388 (described by N. Datta et al, J. Gen. Microbiol., *72*, 349, (1972)) and R7K.

NB. Plasmid pACYC 184 is described by A. C. Y. Chang et al, J. Bacteriol., *134*, 1141—56, (1978).

We have found that the *gdh* gene of *E. coli* can be carried on a small easily manipulated DNA fragment having at its ends target sites for the *SalI* restriction enzyme. This enables the fragment to be conveniently transferred from one plasmid containing it to another by cutting the DNAs of the fragments with *SalI*. The cut DNAs are then mixed and treated with a ligase. For such a step the receiving plasmid requires a *SalI* target either as a normal genotypic feature of the plasmid or imparted to it by an associated transposon or phage.

In one embodiment of the invention a gene, e.g. the *gdh* gene is transferred into a recipient microorganism using a multicopy number plasmid. A multicopy number plasmid is one which, when introduced into a microorganism is capable of replicating itself a significant number of times suitably to produce substantially more than 3 copies of itself per cell, often 8 or 10, preferably at least 20 copies per cell and particularly 20 to 30 copies per cell. By contrast a "single copy" number plasmid is one which replicates itself in a controlled way such that it resides in the cell as 1 to 3 copies. Using a multicopy plasmid a gene such as the *gdh* gene may be introduced into a cell more intensively i.e. the replication of the plasmid produces a substantial number of copies of the gene carrying DNA within the cell. Specifically in the instance of the *gdh* gene introduced into a methanol-utilizing bacterium such as *Methylophilus methylotrophus* it increases the amount of GDH enzyme in the microorganism cells and facilitates establishment therein of an efficient pathway for ammonia assimilation by the GDH pathway. The use of multicopy plasmids has important consequences. A preferred multicopy plasmid for transferring *gdh* gene into *Methylophilus methylotrophus* is R300B.

Preferably when a microorganism is modified by introduction of the *gdh* gene thereby enabling it to assimilate ammonia by the GDH pathway, it is also modified to prevent it using the alternative GS/GUS pathway thereby enabling it to make the most efficient use of the GDH pathway. The GS/GUS pathway can be blocked by ensuring that the microorganism lacks the *glt* gene.

Microorganisms lacking the *glt* gene can be obtained by forming mutants of the original strain e.g. of *Methylophilus methylotrophus* and selecting mutants which have lost this gene. Two methods of selection by (a) temperature and (b) ammonia growth are described in Example 1. Mutants can be produced by standard techniques, for example by treatment with a physical mutagen such as gamma, X or ultra-violet radiation or a chemical mutagen such as N-methyl-$N^1$-nitro-N-nitroso guanidine, nitrous acid, a methane-sulphonate ester (e.g. methyl or ethyl), 5-bromo-uridine, 5-bromouracil, 2-amino-purine or nitrogen mustard or a biological mutagen such as Mu-phage. Treatment to produce a deletion mutant is preferred. The mutant normally remains capable of the GS catalysed reaction.

The invention is illustrated by the following examples:—

Example 1

Transfer of *E. coli gdh* gene into *Methylophilus methylotrophus* strain

The *E. coli gdh* gene was transferred into *Methylophilus methylotrophus* strain NCIB No. 10515 by a method comprising the five steps described below.

1. Development of vector

To generate derivatives of plasmid RP4 carrying the *E. coli gdh* gene the following *E. coli* strains were constructed:—

E401—donor strain for *gdh* gene, with the relevant genotypic features:

*glt* B, *gdh*$^+$, (Mu *cts*): RP4

E412—recipient strain for mating with E401, derived from *E. coli* strain PA340 (alternatively known as CB100), with the relevant genotypic features:

*glt* B, *gdh, rif*$^r$, (Mu *cts*), *rec* A.

The *glt* B and *gdh* mutations render E412 glutamate dependent and in a mating with *E401* only *gdh* gene acquisition will give glutamate independence. The *rec* A mutation renders E412 recombination deficient.

The E401 cells were grown overnight at 37°C to induce Mu *cts* mediated mobilisation of segments of the E401 chromosome to plasmid RP4. Cells from this overnight culture were then mated with E412 cells grown at 30°C. Matings were performed non-selectively by overnight growth at 30°C on L agar plates. Dilutions of cells recovered from the mating plates and control plates (on which donor and recipient cells had been grown separately) were then plated out and selections made for glutamate independence (i.e. the ability to grow using ammonia as nitrogen source), rifampicin (*rif*$^r$) (a selection for recipient based cells) and kanamycin resistance (*Km*$^r$) (a marker borne by the RP4 plasmid).

The cells thus selected were E412 cells with the relevant genotypic features:

*glt* B, *gdh*$^+$ (Mu *cts*): RP4; i.e. cells containing an RP4 plasmid with Mu phage and *gdh* gene attached—RP4 prime *gdh* (RP4'*gdh*).

2. Transfer of *gdh* gene to small plasmid

Plasmid RP4'*gdh* is a large plasmid carrying much unknown DNA and giving problems on transfer and with DNA purification. The *gdh* gene was therefore transferred as follows to the smaller plasmid pACYC184. pACYC184 was selected because (a) it contains a gene specifying chloramphenicol resistance (*Cm*$^r$)—a marker lacking in RP4- and, (b) it has targets for the restriction enzymes *HindIII, BamHI* and *SalI* located within the DNA of the tetracycline resistance gene (*Tc*$^r$) and therefore insertion of "foreign" DNA into these restriction sites on plasmid pACYC184 will inactivate the *Tc*$^r$ gene.

The DNA of plasmid RP4'*gdh* and the DNA of plasmid pACYC184 were both cut with *SalI* restriction enzyme. The two cut DNAs were mixed and a DNA ligase was added. The DNA thus produced was introduced by transformation into *E. coli* strain PA340 (relevant genotype features: *glt*B, *gdh*). A selection was then made for cells exhibiting glutamate independence, chloramphenicol resistance (*Cm*$^r$) and tetracycline sensitivity (*Tc*$^s$). Such cells contain pACYC184 plasmids whose *Tc*$^r$ genes have been cut and fragments of RP4'*gdh* DNA inserted thereinto. It should be noted that pACYC184 is a multicopy plasmid and that when inserted into a cell it will replicate to produce numerous copies. Hence cultures of selected cells produced cells containing numerous copies of the *gdh* gene.

3. Transfer of *gdh* gene to plasmid transferable to NCIB No. 10515

The plasmid pACYC184/*gdh* is not directly transferable into strain NCIB No. 10515. In this plasmid the DNA fragment containing *gdh* is located between targets for restriction enzyme *SalI*. *gdh* fragments were transferred to two alternative plasmids which can be transferred to strain NCIB No. 10515 and which will replicate therein. These plasmids were:—

*pRP301*—a single copy P group plasmid which can transfer itself into strain NCIB No. 10515, and

*R300B*—a multicopy plasmid which can be mobilised into strain NCIB No. 10515 by use of plasmid RP4.

Transposon Tn 5 was transposed into plasmid R300B to give pTB70 as it was required (a) to provide a *SalI* restriction enzyme target on this plasmid which does not have such a target (Molec. Gen. Genet., *171*, 7—13, 1979) and (b) to provide a kanamycin resistance (*Km*$^r$) for the plasmid.

The pACYC184 *gdh* plasmid DNA was cut with *SalI* restriction enzyme and, in separate experiments, was mixed with pRP301 and pTB70 plasmid DNA similarly cut with *SalI* restriction enzyme. In both experiments the resulting DNA mixtures were treated with DNA ligase. The DNAs thus produced in the two experiments were separately introduced by transformation into *E. coli* strain PA340 (relevant genotypic features: *glt*B, *gdh*). Selections were then made in the case of pTB70 for cells exhibiting glutamate independence and kanamycin resistance (*Km*$^r$). These strains were then screened for chloramphenicol sensitivity (*Cm*$^s$). In the case of pRP301 selections were made for cells exhibiting glutamate independence and ampicillin resistance (*amp*) and these strains were screened for tetracycline sensitivity.

4. Transfer of *gdh* gene into strain NCIB No. 10515

In step 3 were produced two alternative modified *E. coli* strains containing respectively plasmid pRP301/*gdh* and plasmid pTB70:*gdh*. In separate experiments these two strains were used as alternative means to transfer the *gdh* into cells of the strain NCIB No. 10515.

Transfer using pTB70:*gdh*

Plasmid R300B cannot mobilise itself into cells of strain NCIB No. 10515, i.e. R300B is not transferred from a strain carrying R300B to another strain. This difficulty was overcome by inserting into the *E. coli* cells

containing pTB70:*gdh* an additional plasmid which can mobilise itself into cells of NCIB No. 10515. The extra plasmid inserted was RP4 whose properties enable it to provide a method for the transfer of plasmids such as plasmid pTB70:*gdh* from one cell to another. *E. coli* cells were therefore prepared containing the plasmids pTB70:*gdh* and RP4. A streptomycin resistance marker (*Sm*$^r$) is carried by plasmid R300B. NCIB No. 10515 lacks this resistance. This streptomycin resistance was then used to select for transfer of pTB70:*gdh* to NCIB 10515.

A culture medium suitable for strain NCIB No. 10515 but including streptomycin was prepared and placed upon a petri dish which was divided, one half being used as a control whilst cells of NCIB No. 10515 were placed on the other half. Both parts were streaked with a culture containing *E. coli* cells modified as described above. On the control part of the dish *E. coli* cells did not survive. On the other side, whilst the *E. coli* cells did not survive, it was possible for DNA material to be transmitted from them to cells of NCIB No. 10515. This enabled NCIB No. 10515 cells which had received DNA containing pTB70:*gdh* with associated (*Sm*$^r$) to survive, i.e. selection was made for (*Sm*$^r$).

The resulting bacterial strain—*Modification 2*—was a modification of NCIB No. 10515 with the relevant genotypic features: *glt*$^+$, *gdh*$^+$, pTB70, *Sm*$^r$.

Transfer using pRP301/*gdh*

This plasmid was also transferred into strain NCIB No. 10515 using suitable methods to give *Modification 1*—a modification of NCIB No. 10515 with the relevant genotypic features: *glt*$^+$, *gdh*$^+$, pRP301.

Thus both Modifications 1 and 2 possess both of the alternative pathways for ammonia assimilation.

5. Transfer of *gdh* gene into *glt* NCIB No. 10515

To obtain an NCIB No. 10515 strain relying entirely upon the GDH pathway for ammonia assimilation it was necessary first to obtain mutants of NCIB No. 10515 lacking the *glt* gene. Two methods were employed.

(a) Temperature sensitive mutants—NCIB No. 10515 cells were treated with N-methyl-N$^1$-nitro-N-nitroso guanidine and the resulting variety of mutants was examined at 30°C and 37°C. A total of 550 temperature sensitive mutants, i.e. ones capable of growth at 30°C but not at 37°C, were collected. These were examined to find mutants in which the *glt* gene product had become temperature sensitive. The mutants were mated as described in 4 above with *E. coli* cells containing plasmids pTB70:*gdh* and RP4. Four mutants were, after this mating, capable of growth at 37°C. This implied that in these four cases the mutant cells were ones in which the *glt* gene product had become temperature sensitive but that, having acquired pTB70:*gdh* from the *E. coli* cells, growth at 37°C became possible. This result was confirmed by incorporating into separate samples of each of the original mutants pTB70:*gdh* and pTB70 (using the method of 4 above) and showing that only transfer of the former plasmid allowed growth at 37°C, and thus that growth was due to a function coded for on the cloned fragment of *E. coli* DNA carrying the *gdh* gene. Furthermore, these temperature sensitive mutants carrying pTB70:*gdh* were unable to form colonies at 37°C on plates supplemented with concentrations of ammonia at 0.07 mM or less, on which NCIB No. 10515 can form colonies. This implies that these cells are using the *gdh* gene product for growth since cells using the *glt* gene product can grow on much lower concentrations of ammonia than cells using *gdh*. Finally it was confirmed by enzyme assays on cells grown at 37°C that these temperature sensitive mutants carrying pTB70:*gdh* lacked detectable GUS activity, but had GDH activity, whereas NCIB No. 10515 had GUS activity but lacked detectable GDH activity.

One of the four temperature sensitive mutants modified by inclusion of plasmid pTB70:*gdh* was deposited at NCIB as NCIB No. 11585 (Modification 3) on 5 March 1980.

Further modifications were prepared by incorporating into temperature sensitive mutants deficient in the *glt* gene product, the plasmid pRP301:*gdh*.

(b) Ammonia growth selected mutants—In separate experiments cells of Modifications 1 and 2 were used as "wild-type" strains. These cells contain both the *gdh* and the *glt* genes. However mutants may occur lacking the *glt* gene. For efficient assimilation of ammonia by the GDH pathway a high concentration of ammonia is desirable. The alternative GS/GUS pathway functions well at low ammonia concentrations. Hence cells of Modifications 1 and 2, having both pathways should function well at both high and low concentrations of ammonia. In the experiments therefore cells carrying the pTB70:*gdh* plasmid were grown on media containing (a) >30 mM ammonia and (b) <.07 mM and cells were selected which were unable to grow on the medium containing <.07 mM. In this way mutants of strain NCIB No. 10515 were obtained which were *gdh*$^+$ and *glt* (some derived from Modification 1 and some from Modification 2).

Example 2

Production of SCP using Modification 3 (NCIB No. 11585)

The organisms designated NCIB No. 10515 and NCIB No. 11585, the latter using GDH activity rather than GUS/GS, were grown in continuous culture in a methanol/mineral salts medium designed to support 30 gms/l dry weight of cells growing at a dilution rate of 0.2 hr$^{-1}$. Temperature was controlled at 37°C±1°, pH 6.8—7.0 by the demand addition of ammonia gas. The cultures were supplied with air as oxygen source.

Modification 3 cultures were grown in the presence of not less than 50 mM ammonia in order to saturate GDH.

Carbon conversions of methanol carbon to organic cellular carbon were determined by total carbon analysis. Results gave a growth yield improvement of 4 to 7% for NCIB No. 11585 over NCIB No. 10515.

**Claims for the Contracting States: BE, CH, DE, FR, GB, IT, LI, LU, NL, SE**

1. A method for making a genetically modified microorganism by incorporating DNA material comprising a *gdh* gene into a microorganism deficient in this gene, so that expression of the *gdh* gene product, i.e. the active GDH enzyme, occurs in the previously *gdh* gene-deficient microorganism, which method comprises the steps of (a) introducing into a first microorganism having the *gdh* gene a plasmid vector for said gene, and (b) transferring said gene on said vector into a second microorganism.

2. A method for making a genetically modified microorganism by transferring DNA material comprising the *gdh* gene from a donor microorganism having said gene to a recipient microorganism deficient therein wherein the DNA material is transferred into the recipient microorganism on a plasmid vector formed from a multicopy plasmid which plasmid, when introduced into the recipient microorganism, is capable of replicating itself to produce more than 3 copies of itself per cell.

3. A method for making a genetically modified microorganism by transferring DNA material comprising the *gdh* gene from a donor microorganism having said gene to a recipient microorganism deficient therein which comprises a step wherein the *gdh* gene containing DNA material is transferred on a DNA fragment having at its ends target sites for the *SalI* restriction enzyme.

4. A method according to claim 1 wherein the second microorganism is an intermediate and the *gdh* gene is transferred via one or more intermediate microorganisms from the first microorganism to the *gdh* gene-deficient microorganism.

5. A method according to claim 2 which comprises a plurality of steps involving at least one intermediary microorganism during transfer of the DNA material from the donor to the recipient microorganism.

6. A method according to claim 3 wherein the DNA fragment transferred is transferred on a plasmid vector formed from a multicopy plasmid.

7. A method according to any one of the preceding claims wherein a *gdh* gene is transferred into a gram-negative bacterium lacking this gene.

8. A method according to claim 7 wherein the gram-negative bacterium is a strain of the species *Methylophilus methylotrophus*.

9. A method according to claim 8 wherein the strain is *Methylophilus methylotrophus* NCIB No. 10515.

10. A method according to any one of the preceding claims which comprises a step in which the *glt* gene product is inactivated by mutation in *gdh* gene-deficient microorganisms.

11. A method according to any one of the preceding claims wherein the *gdh* gene containing DNA material is obtained from a strain of *E. coli*.

12. A method according to any one of the preceding claims wherein the *gdh* gene containing DNA material is transferred into the *gdh* gene deficient microorganism on a plasmid vector formed from an Inc Q or Inc W group plasmid.

13. Genetically modified microorganisms produced by a method according to any one of the preceding claims which are strains of the species *Methylophilus methylotrophus* genetically modified by incorporation of DNA material comprising a *gdh* gene.

14. The microorganism *Methylophilus methylotrophus* strain NCIB No. 11585 and derivatives therefrom.

15. A process for the production of single cell protein and/or an amino acid wherein a gram-negative bacterium genetically modified by incorporation of DNA material comprising a *gdh* gene and produced by a method according to any one of claims 1 to 12 is aerobically cultured in an aqueous medium containing methanol as a source of assimilable carbon and ammonia and/or ammonium ion at a concentration sufficient to maintain the GDH reaction mechanism and the single cell protein, a composition containing it and/or an amino acid is recovered from the culture medium.

16. A process according to claim 15 wherein the genetically modified microorganism is a genetically modified strain of the species *Methylophilus methylotrophus*.

17. A process according to claim 16 wherein the genetically modified microorganism is *Methylophilus methylotrophus* strain NCIB No. 11585 or derivatives therefrom.

18. Dead cells of microorganisms according to claim 13 or claim 14.

19. A foodstuff for animal or human consumption containing dead cells according to claim 18.

**Claims for the Contracting State: AT**

1. A method for making a genetically modified microorganism by incorporating DNA material comprising a *gdh* gene into a microorganim deficient in this gene, so that expression of the *gdh* gene product, i.e. the active GDH enzyme, occurs in the previously *gdh* gene-deficient microorganism, which

method comprises the steps of (a) introducing into a first microorganism having the *gdh* gene a plasmid vector for said gene, and (b) transferring said gene on said vector into a second microorganism.

2. A method for making a genetically modified microorganism by transferring DNA material comprising the *gdh* gene from a donor microorganism having said gene to a recipient microorganism deficient therein wherein the DNA material is transferred into the recipient microorganism on a plasmid vector formed from a multicopy plasmid which plasmid, when introduced into the recipient microorganism, is capable of replicating itself to produce more than 3 copies of itself per cell.

3. A method for making a genetically modified microorganism by transferring DNA material comprising the *gdh* gene from a donor microorganism having said gene to a recipient microorganism deficient therein which comprises a step wherein the *gdh* gene containing DNA material is transferred on a DNA fragment having at its ends target sites for the *SalI* restriction enzyme.

4. A method according to claim 1 wherein the second microorganism is an intermediate and the *gdh* gene is transferred via one or more intermediate microorganisms from the first microorganism to the *gdh* gene-deficient microorganism.

5. A method according to claim 2 which comprises a plurality of steps involving at least one intermediary microorganism during transfer of the DNA material from the donor to the recipient microorganism.

6. A method according to any one of the preceding claims wherein the microorganism is a strain of the species *Methylophilus methylotrophus*.

7. A method according to claim 6 wherein the strain is *Methylophilus methylotrophus* NCIB No. 10515.

8. A method according to any one of the preceding claims which comprises a step in which the *glt* gene product is inactivated by mutation in *gdh* gene-deficient microorganisms.

9. A process for the production of single cell protein and/or an amino acid wherein a gram-negative bacterium genetically modified by incorporation of DNA material comprising a *gdh* gene and produced by a method according to any one of claims 1 to 8 is aerobically cultured in an aqueous medium containing methanol as a source of assimilable carbon and ammonia and/or ammonium ion at a concentration sufficient to maintain the GDH reaction mechanism and the single cell protein, a composition containing it and/or an amino acid is recovered from the culture medium.

10. A process according to claim 9 wherein the genetically modified microorganism is a genetically modified strain of the species *Methylophilus methylotrophus*.

**Patentansprüche für die Vertragsstaaten: BE, CH, DE, FR, GB, IT, LI, LU, NL, SE**

1. Verfahren zur Herstellung eines genetisch modifizierten Mikroorganismus durch Einbau von DNA-Material, das ein *gdh*-Gen enthält, in einen Mikroorganismus, dem es an diesem Gen mangelt, so daß in dem Mikroorganismus, dem es zuvor an dem *gdh*-Gen mangelte, eine Induktion der Bildung des Produkts des *gdh*-Gens, d.h. des aktiven GDH-Enzyms, eintritt, wobei dieses Verfahren die Schritte (a) der Einführung eines Plasmid-Vektors für das *gdh*-Gen in einen ersten Mikroorganismus, der dieses Gen aufweist, und (b) der Übertragung bzw. des Transfers dieses Gens auf dem Vektor in einen zweiten Mikroorganismus enthält.

2. Verfahren zur Herstellung eines genetisch modifizierten Mikroorganismus durch Übertragung von DNA-Material, in dem das *gdh*-Gen enthalten ist, von einem Donor-Mikroorganismus, der dieses Gen aufweist, auf einen Empfänger-Mikroorganismus, dem es daran mangelt, bei dem das DNA-Material in den Empfänger-Mikroorganismus auf einem Plasmid-Vektor, der aus einem Mehrfachkopie-Plasmid gebildet ist, übertragen wird, wobei dieses Plasmid, wenn es in den Empfänger-Mikroorganismus eingeführt worden ist, imstande ist, sich unter Erzeugung von mehr als 3 Kopien von sich selbst pro Zelle zu replizieren.

3. Verfahren zur Herstellung eines genetisch modifizierten Mikroorganismus durch Übertragung von DNA-Material, in dem das *gdh*-Gen enthalten ist, von einem Donor-Mikroorganismus, der dieses Gen aufweist, auf einen Empfänger-Mikroorganismus, dem es daran mangelt, wobei das Verfahren einen Schritt enthält, in dem das DNA-Material, in dem das *gdh*-Gen enthalten ist, auf einem DNA-Fragment, das an seinen Enden Erkennungsstellen für das Restriktionsenzym *SalI* aufweist, übertragen wird.

4. Verfahren nach Anspruch 1, bei dem der zweite Mikroorganismus ein Zwischenglied ist und das *gdh*-Gen über einen oder mehr als einen als Zwischenglied dienenden Mikroorganismus von dem ersten Mikroorganismus auf den Mikroorganismus, dem es an dem *gdh*-Gen mangelt, übertragen wird.

5. Verfahren nach Anspruch 2, das eine Vielzahl von Schritten enthält, an denen während der Übertragung des DNA-Materials von dem Donor- auf den Empfänger-Mikroorganismus mindestens ein als Zwischenglied dienender Mikroorganismus beteiligt ist.

6. Verfahren nach Anspruch 3, bei dem das übertragene DNA-Fragment auf einem Plasmid-Vektor, der aus einem Mehrfachkopie-Plasmid gebildet ist, übertragen wird.

7. Verfahren nach einem der vorhergehenden Ansprüche, bei dem ein *gdh*-Gen in eine gramnegative Bakterie, der es an diesem Gen mangelt, übertragen wird.

8. Verfahren nach Anspruch 7, bei dem die gramnegative Bakterie ein Stamm der Art *Methylophilus methylotrophus* ist.

9. Verfahren nach Anspruch 8, bei dem der Stamm *Methylophilus methylotrophus* NCIB Nr. 10515 ist.

10. Verfahren nach einem der vorhergehenden Ansprüche, das einen Schritt enthält, in dem das Produkt des *glt*-Gens durch Mutation in Mikroorganismen, denen es an *gdh*-Gen mangelt, inaktiviert wird.

11. Verfahren nach einem der vorhergehenden Ansprüche, bei dem das DNA-Material, in dem das *gdh*-Gen enthalten ist, aus einem Stamm von *E. coli* erhalten wird.

12. Verfahren nach einem der vorhergehenden Ansprüche, bei dem das DNA-Material, in dem das *gdh*-Gen enthalten ist, auf einem Plasmid-Vektor, der aus einem Plasmid der Inc Q- oder Inc W-Gruppe gebildet ist, in den Mikroorganismus, dem es an dem *gdh*-Gen mangelt, übertragen wird.

13. Genetisch modifizierte Mikroorganismen, die durch ein Verfahren nach einem der vorhergehenden Ansprüche hergestellt sind und bei denen es sich um Stämme der Art *Methylophilus methylotrophus* handelt, die durch Einbau von DNA-Material, das ein *gdh*-Gen enthält, genetisch modifiziert wurden.

14. Stamm NCIB Nr. 11585 des Mikroorganismus *Methylophilus methylotrophus* und Abkömmlinge davon.

15. Verfahren zur Herstellung von Einzellerprotein und/oder einer Aminosäure, bei dem eine gramnegative Bakterie, die durch Einbau von DNA-Material, das ein *gdh*-Gen enthält, genetisch modifiziert und durch ein Verfahren nach einem der Ansprüche 1 bis 12 hergestellt wurde, in einem wäßrigen Medium, das Methanol als Quelle assimilierbaren Kohlenstoffs und Ammoniak und/oder Ammoniumionen in einer zur Aufrechterhaltung des GDH-Reaktionsmechanismus ausreichenden Konzentration enthält, aerob gezüchtet wird und das Einzellerprotein, eine Masse, in der es enthalten ist, und/oder eine Aminosäure aus dem Nährmedium gewonnen wird.

16. Verfahren nach Anspruch 15, bei dem der genetisch modifizierte Mikroorganismus ein genetisch modifizierter Stamm der Art *Methylophilus methylotrophus* ist.

17. Verfahren nach Anspruch 16, bei dem der genetisch modifizierte Mikroorganismus der Stamm NCIB Nr. 11585 von *Methylophilus methylotrophus* oder ein Abkömmling davon ist.

18. Leblose Zellen von Mikroorganismen nach Anspruch 13 oder 14.

19. Leblose Zellen nach Anspruch 18 enthaltendes Futter- bzw. Nahrungsmittel für den Verbrauch durch Tiere oder Menschen.

**Patentansprüche für den Vertragsstaat: AT**

1. Verfahren zur Herstellung eines genetisch modifizierten Mikroorganismus durch Einbau von DNA-Material, das ein *gdh*-Gen enthält, in einen Mikroorganismus, dem es an diesem Gen mangelt, so daß in dem Mikroorganismus, dem es zuvor an dem *gdh*-Gen mangelte, eine Induktion der Bildung des Produkts des *gdh*-Gens, d.h. des aktiven GDH-Enzyms, eintritt, wobei dieses Verfahren die Schritte (a) der Einführung eines Plasmid-Vektors für das *gdh*-Gen in einen ersten Mikroorganismus, der dieses Gen aufweist, und (b) der Übertragung bzw. des Transfers dieses Gens auf dem Vektor in einen zweiten Mikroorganismus enthält.

2. Verfahren zur Herstellung eines genetisch modifizierten Mikroorganismus durch Übertragung von DNA-Material, in dem das *gdh*-Gen enthalten ist, von einem Donor-Mikroorganismus, der dieses Gen aufweist, auf einen Empfänger-Mikroorganismus, dem es daran mangelt, bei dem das DNA-Material in den Empfänger-Mikroorganismus auf einem Plasmid-Vektor, der aus einem Mehrfachkopie-Plasmid gebildet ist, übertragen wird, wobei dieses Plasmid, wenn es in den Empfänger-Mikroorganismus eingeführt worden ist, imstande ist, sich unter Erzeugung von mehr als 3 Kopien von sich selbst pro Zelle zu replizieren.

3. Verfahren zur Herstellung eines genetisch modifizierten Mikroorganismus durch Übertragung von DNA-Material, in dem das *gdh*-Gen enthalten ist, von einem Donor-Mikroorganismus, der dieses Gen aufweist, auf einen Empfänger-Mikroorganismus, dem es daran mangelt, wobei das Verfahren einen Schritt enthält, in dem das DNA-Material, in dem das *gdh*-Gen enthalten ist, auf einem DNA-Fragment, das an seinen Enden Erkennungsstellen für das Restriktionsenzym *SalI* aufweist, übertragen wird.

4. Verfahren nach Anspruch 1, bei dem der zweite Mikroorganismus ein Zwischenglied ist und das *gdh*-Gen über einen oder mehr als einen als Zwischenglied dienenden Mikroorganismus von dem ersten Mikroorganismus auf den Mikroorganismus, dem es an dem *gdh*-Gen mangelt, übertragen wird.

5. Verfahren nach Anspruch 2, das eine Vielzahl von Schritten enthält, an denen während der Übertragung des DNA-Materials von dem Donor- auf den Empfänger-Mikroorganismus mindestens ein als Zwischenglied dienender Mikroorganismus beteiligt ist.

6. Verfahren nach einem der vorhergehenden Ansprüche, bei dem der Mikroorganismus ein Stamm der Art *Methylophilus methylotrophus* ist.

7. Verfahren nach Anspruch 6, bei dem der Stamm *Methylophilus methylotrophus* NCIB Nr. 10515 ist.

8. Verfahren nach einem der vorhergehenden Ansprüche, das einen Schritt enthält, in dem das Produkt des *glt*-Gens durch Mutation in Mikroorganismen, denen es an *gdh*-Gen mangelt, inaktiviert wird.

9. Verfahren zur Herstellung von Einzellerprotein und/oder einer Aminosäure, bei dem eine gramnegative Bakterie, die durch Einbau von DNA-Material, das ein *gdh*-Gen enthält, genetisch modifiziert und durch ein Verfahren nach einem der Ansprüche 1 bis 8 hergestellt wurde, in einem wäßrigen Medium, das Methanol als Quelle assimilierbaren Kohlenstoffs und Ammoniak und/oder Ammoniumionen in einer zur Aufrechterhaltung des GDH-Reaktionsmechanismus ausreichenden Konzentration enthält, aerob

gezüchtet wird und das einzellerprotein, eine Masse, in der es enthalten ist, und/oder eine Aminosäure aus dem Nährmedium gewonnen wird.

10. Verfahren nach Anspruch 9, bei dem der genetisch modifizierte Mikroorganismus ein genetisch modifizierter Stamm der Art *Methylophilus methylotrophus* ist.

**Revendications Pour les Etats Contractants: BE, CH, DE, FR, GB, IT, LI, LU, NL, SE**

1. Procédé de production d'un micro-organisme génétiquement modifié par incorporation de matériel de DNA comprenant un gène *gdh* dans un micro-organisme déficient en ce gène, de façon que l'expression du produit du gène *gdh*, c'est-à-dire l'enzyme GDH active, ait lieu dans le micro-organisme antérieurement déficient en le gène *gdh*, lequel procédé comprend les stades (a) d'introduire dans un premier micro-organisme comprenant le gène *gdh* un plasmide vecteur pour ce gène et (b) de transférer ce gène sur ce vecteur dans un second microorganisme.

2. Procédé de production d'un micro-organisme génétiquement modifié par transfert de matériel de DNA comprenant le gène *gdh* depuis un micro-organisme donneur comprenant ce gène jusqu'à un micro-organisme receveur déficient en celui-ci, dans lequel le matériel de DNA est transféré dans le micro-organisme receveur sur un plasmide vecteur formé à partir d'un plasmide à copies multiples, lequel plasmide, lorsqu'il est introduit dans le micro-organisme receveur, est capable de s'y répliquer pour produire plus de 3 copies de lui-même par cellule.

3. Procédé de production d'un micro-organisme génétiquement modifié par transfert de matériel de DNA comprenant le gène *gdh* depuis un micro-organisme donneur comprenant ce gène à un micro-organisme receveur déficient en celui-ci, qui comprend un stade dans lequel le matériel de DNA contenant le gène *gdh* est transféré sur un fragment de DNA comportant à ses extrémités des sites cibles pour l'enzyme de restriction *SalI*.

4. Procédé suivant la revendication 1, dans lequel le second micro-organisme est un intermédiaire et le gène *gdh* est transféré à l'intervention d'un ou plusieurs micro-organismes intermédiaires depuis le premier micro-organisme jusqu'au micro-organisme déficient en le gène *gdh*.

5. Procédé suivant la revendication 2, qui comprend plusieurs stades mettant en jeu au moins un micro-organisme intermédiaire pendant le transfert du matériel de DNA du micro-organisme donneur au micro-organisme receveur.

6. Procédé suivant la revendication 3, dans lequel le fragment de DNA transféré est transféré sur un plasmide vecteur formé à partir d'un plasmide à copies multiples.

7. Procédé suivant l'une quelconque des revendications précédentes, dans lequel un gène *gdh* est transféré dans une bactérie Gram-négative exempte de ce gène.

8. Procédé suivant la revendication 7, dans lequel la bactérie Gram-négative est une souche de l'espèce *Methylophilus methylotrophus*.

9. Procédé suivant la revendication 8, dans lequel la souche est *Methylophilus methylotrophus* NCIB No. 10515.

10. Procédé suivant l'une quelconque des revendications précédentes, qui comprend un stade au cours duquel le produit du gène *glt* est inactivé par mutation dans des micro-organismes déficients en le gène *gdh*.

11. Procédé suivant l'une quelconque des revendications précédentes, dans lequel le matériel de DNA comprenant le gène *gdh* est obtenu à partir d'une souche d'*E. coli*.

12. Procédé suivant l'une quelconque des revendications précédentes, dans lequel le matériel de DNA contenant le gène *gdh* est transféré dans le microorganisme déficient en le gène *gdh* sur un plasmide vecteur formé au départ d'un plasmide du groupe Inc Q ou Inc W.

13. Micro-organismes génétiquement modifiés produits par un procédé suivant l'une quelconque des revendications précédentes qui sont des souches de l'espèce *Methylophilus methylotrophus* génétiquement modifiées par incorporation de matériel de DNA comprenant un gène *gdh*.

14. Le micro-organisme *Methylophilus methylotrophus* souche NCIB No. 11585 et ses dérivés.

15. Procédé de production de protéine d'origine unicellulaire et/ou d'un aminoacide dans lequel une bactérie Gram-négative génétiquement modifiée par incorporation de matériel de DNA comprenant un gène *gdh* et produite par un procédé suivant l'une quelconque des revendications 1 à 12 est cultivée en aérobiose dans un milieu aqueux contenant du méthanol comme source de carbone assimilable et de l'ammoniac et/ou l'ion ammonium en une concentration suffisante pour entretenir le mécanisme de réaction GDH, et la protéine d'origine unicellulaire, une composition la contenant et/ou un aminoacide sont isolés du milieu de culture.

16. Procédé suivant la revendication 15, dans lequel le micro-organisme génétiquement modifié est une souche génétiquement modifiée de l'espèce *Methylophilus methylotrophus*.

17. Procédé suivant la revendication 16, dans lequel le micro-organisme génétiquement modifié est *Methylophilus methylotrophus* souche NCIB No. 11585 ou un dérivé de celle-ci.

18. Cellules mortes de micro-organismes suivant la revendication 13 ou 14.

19. Aliment pour la consommation humaine ou animale contenant des cellules mortes suivant la revendication 18.

**Revendications pour l'Etat Contractant: AT**

1. Procédé de production d'un micro-organisme génétiquement modifié par incorporation de matériel de DNA comprenant un gène *gdh* dans un micro-organisme déficient en ce gène, de façon que l'expression du produit du gène *gdh*, c'est-à-dire l'enzyme GDH active, ait lieu dans le micro-organisme antérieurement déficient en le gène *gdh*, lequel procédé comprend les stades (a) d'introduire dans un premier micro-organisme comprenant le gène *gdh* un plasmide vecteur pour ce gène et (b) de transférer ce gène sur ce vecteur dans un second micro-organisme.

2. Procédé de production d'un micro-organisme génétiquement modifié par transfert de matériel de DNA comprenant le gène *gdh* depuis un micro-organisme donneur comprenant ce gène jusqu'à un micro-organisme receveur déficient en celui-ci, dans lequel le matériel de DNA est transféré dans le micro-organisme receveur sur un plasmide vecteur formé a partir d'un plasmide à copies multiples, lequel plasmide, lorsqu'il est introduit dans le micro-organisme receveur, est capable de s'y répliquer pour produire plus de 3 copies de lui-même par cellule.

3. Procédé de production d'un micro-organisme génétiquement modifié par transfert de matériel de DNA comprenant le gène *gdh* depuis un micro-organisme donneur comprenant ce gène à un microorganisme receveur déficient en celui-ci, qui comprend un stade dans lequel le matériel de DNA contenant le gène *gdh* est transféré sur un fragment de DNA comportant à ses extrémités des sites cibles pour l'enzyme de restriction *SalI*.

4. Procédé suivant la revendication 1, dans lequel le second micro-organisme est un intermédiaire et le gène *gdh* est transféré à l'intervention d'un ou plusieurs micro-organismes intermédiaires depuis le premier micro-organisme jusqu'au micro-organisme déficient en le gène *gdh*.

5. Procédé suivant la revendication 2, qui comprend plusieurs stades mettant en jeu au moisn un micro-organisme intermédiaire pendant le transfert du matériel de DNA du micro-organisme donneur au micro-organisme receveur.

6. Procédé suivant l'une quelconque des revendications précédentes, dans lequel le micro-organisme est une souche de l'espèce *Methylophilus methylotrophus*.

7. Procédé suivant la revendication 6, dans lequel la souche est *Methylophilus methylotrophus* NCIB No. 10515.

8. Procédé suivant l'une quelconque des revendications précédentes, qui comprend un stade au cours duquel le produit du gène *glt* est inactivé par mutation dans des micro-organismes déficients en le gène *gdh*.

9. Procédé de production de protéine d'origine unicellulaire et/ou d'un aminoacide, dans lequel une bactérie Gram-négative génétiquement modifiée par incorporation de matériel de DNA comprenant un gène *gdh* et produite par un procédé suivant l'une quelconque des revendications 1 à 8 est cultivée en aérobiose dans un milieu aqueux contenant du méthanol comme source de carbone assimilable et de l'ammoniac et/ou l'ion ammonium en une concentration suffisante pour entretenir le mécanisme de réaction GDH, et la protéine d'origine unicellulaire, une composition la contenant et/ou un aminoacide sont isolés du milieu de culture.

10. Procédé suivant la revendication 9, dans lequel le micro-organisme génétiquement modifié est une souche génétiquement modifiée de l'espèce *Methylophilus methylotrophus*.